# EUROPÄISCHE PATENTSCHRIFT

(11) **EP 0 940 384 B1**
(45) Veröffentlichungstag und Bekanntmachung des Hinweises auf die Patenterteilung: **12.06.2002**
(21) Anmeldenummer: 99102222.9
(22) Anmeldetag: 04.02.1999
(51) Int. Cl.: C07C 45/82, C07C 45/85, C07C 45/67, C07C 49/293

(54) **Verfahren zur Herstellung von Cyclopropylalkylketonen**
Process for the preparation of cyclopropylalkylketones
Procédé pour la préparation de cyclopropylalkylcétones

(30) Priorität: 06.03.1998 DE 19809775
(43) Veröffentlichungstag der Anmeldung: 08.09.1999
(73) Patentinhaber: Degussa AG, 40474 Düsseldorf (DE)
(72) Erfinder: Korell, Michael, Dr., 44801 Bochum (DE); Kleemiss, Wolfgang, Dr., 45721 Haltern (DE); Kaufhold, Manfred, Dr., 45770 Marl (DE); Jegelka, Udo, Dr., 45665 Recklinghausen (DE)

(56) Entgegenhaltungen:
- EP-A- 0 864 556
- US-A- 5 254 739

## Beschreibung

Die Erfindung betrifft ein neues Verfahren zur Herstellung von Cyclopropylalkylketonen mit C₁- bis C₄-Alkylgruppen aus 3-Acyltetrahydrofuran-2-onen.

Cyclopropylketone sind wichtige Zwischenprodukte zur Herstellung von Pharmaka und Agrochemikalien.

Ihre Herstellung erfolgt nach dem Schema:

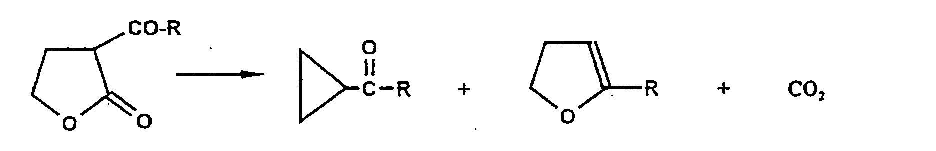

Als Nebenprodukt entstehen also auch 4,5-Dihydro-2-alkylfurane und Kohlendioxid.

Verfahren zur Herstellung der Cyclopropylalkylketone sind nach EP-A-0 552 586 und EP-A-0 725 066 bekannt. Dabei wird 3-Acyltetrahydrofuran-2-on mit Alkalihalogenid bei 160 bis 220 °C umgesetzt, worauf Cyclopropylalkylketon und das Nebenprodukt 4,5-Dihydro-2-alkylfuran destilliert und kondensiert werden.

Bei der Herstellung von Cyclopropylmethylketon (CPMK) aus 3-Acetyltetrahydrofuran-2-on bzw. Acetylbutyrolacton (ABL) entsteht 4,5-Dihydro-2-methylfuran (DHMF) in Mengen von meist 5 bis 30 %, bezogen auf die Summe aus CPMK und DHMF.

Um hochreines CPMK zu erhalten, muß das Gemisch aus CPMK und DHMF fraktioniert werden. In der EP-A 864 556 wird daher ein kontinuierliches Verfahren zur destillativen Abtrennung von DHMF beschrieben. Dazu bedarf es aber einer Rektifikationskolonne mit hoher Trennleistung.

DHMF ist außerdem eine Verbindung, die nach Houben-Weyl, Methoden der organischen Chemie Band VI/3, 1965, S. 698, in Gegenwart von Säurespuren mit Nucleophilen unter Freisetzung großer Wärmemengen heftig reagieren kann. Bei der Herstellung von CPMK im Produktionsmaßstab ist daher die Handhabung eines so reaktiven Nebenproduktes wie DHMF unerwünscht. Denn durch unsachgemäßen Umgang mit DHMF oder mit einer DHMF-haltigen Fraktion kann Schaden für Mensch und Umwelt entstehen.

Es bestand daher die Aufgabe, die risikoreichen 4,5-Dihydro-2-alkylfurane auf eine technisch möglichst einfache, unter Sicherheitsaspekten und bezüglich der Aufarbeitung oder Entsorgung der Produkte problemlose, hinreichend wirksame Methode abzutrennen und unschädlich zu machen.

Die Aufgabe wird erfindungsgemäß dadurch gelöst, daß man die bei der Reaktion anfallenden 4,5-Dihydro-2-alkylfurane vor, während oder nach der Destillation der Reaktionsprodukte in einer sauer katalysierten Reaktion mit Alkoholen, Wasser oder wäßrigen Alkoholen bei 10°C bis 80°C umsetzt und dann abtrennt.

Es überrascht, daß auf diese Art 4,5-Dihydro-2-alkylfurane mit hoher Geschwindigkeit weitgehend umgesetzt und problemlos entfernt werden können. Die freiwerdende Reaktionswärme wird durch einen Überschuß an Abfangmedium aus Alkoholen, Wasser oder Alkohol/Wasser-Gemischen aufgenommen und so kontrolliert abgeführt. Dieses ist besonders dann gewährleistet, wenn die Reaktion in homogener Phase durchgeführt wird.

Der Überschuß an Abfangmedium kann beliebig hoch sein. Aus wirtschaftlichen Gründen liegt das Gewichtsverhältnis von 4,5-Dihydro-2-alkylfuran zu Abfangmedium meist bei 1 : 2 bis 1 : 50. Dabei werden Gewichtsverhältnisse von 1 : 5 bis 1 : 30 vorzugsweise eingestellt.

DHMF weist gegenüber Wasser eine besonders hohe Reaktivität auf. Um bei der Umsetzung von DHMF mit Wasser die Ausbildung von zwei Phasen sicher zu vermeiden, werden Mischungen von Wasser mit Alkoholen bevorzugt verwendet. Der Wassergehalt der Mischungen liegt daher oft bei maximal 40 Gew.-% und bevorzugt sogar nur bei maximal 20 Gew.-%. Vorzugsweise werden auch wasserfreie Alkohole eingesetzt.

Geeignete Alkohole sind beispielsweise Ethanol, Isopropanol, n-Propanol, n-Butanol, Pentanol, Hexanol, 2-Ethylhexanol, Nonanol, Decanol sowie mehrwertige Alkohole wie Ethylenglykol und Glycerin. Vorzugsweise verwendet man Alkohole mit einem Siedepunkt von 100 bis 300 °C.

Bezogen auf die Summe aus Wasser und Alkohol können die zur Umsetzung mit den 4,5-Dihydro-2-alkylfuranen eingesetzten Medien auch bis zu 20 Gew.-% andere organische Lösemittel, wie beispielsweise Ketone, Ether oder Ester, enthalten.

Obwohl bei den Cyclopropylalkylketonen auch Ethyl, Isopropyl und Butyl als Alkylgruppen in Betracht kommen, wird erfindungsgemäß die Methylgruppe bevorzugt. Vorzugsweise wird also ABL zu CPMK und DHMF umgesetzt.

Die Abfangmedien weisen außerdem als Katalysatoren, bezogen auf die Summe aus Wasser und Alkoholen, vorzugsweise bis zu 15 Gew.-% Carbon-, Sulfon- oder Mineralsäuren auf. Besonders bevorzugt liegt der Gehalt bei unter 10 Gew.-%, wobei Anteile von 1 bis 6 Gew.-% ganz besonders bevorzugt werden. Saure Ionenaustauscher und saure Mineralien, wie zum Beispiel Alumosilikate, können ebenfalls eingesetzt werden.

Geeignete Carbonsäuren sind beispielsweise Essigsäure, Propionsäure, Maleinsäure, Fumarsäure, Bernsteinsäure, Adipinsäure, p-Toluolsulfonsäure oder Methansulfonsäure.

Als Mineralsäuren seien hier nur Salzsäure, Schwefelsäure und Phosphorsäure genannt.

Die Umsetzung wird bei Temperaturen von 10 bis 80 °C durchgeführt.

Wird das Abfangmedium, ein nucleophiler Reaktionspartner, vor der Reindestillation zugesetzt, so erhält man bei der Destillation ein Cyclopropylalkylketon ohne eine 4,5-Dihydro-2-alkylfuran enthaltende Vorfraktion.

Nun sind Cyclopropylalkylketone und 4,5-Dihydro-2-alkylfurane und insbesonders CPMK und DHMF relativ leichtflüchtige Verbindungen, die bei der Reaktion in der Praxis nur mit erheblichem Aufwand durch Kondensation quantitativ abgeschieden werden können. Meist wird daher das Abgas, das überwiegend aus Kohlendioxid besteht, auch Cyclopropylalkylketone und 4,5-Dihydro-2-alkylfurane in solchen Mengen enthalten, daß es ohne vorherige Reinigung nicht emittiert werden kann.

Zwar können nach Ullmann's Encyclopedia of Industrial Chemistry, 5th Ed., Vol. B 3, S. 8-7 ff und S. 9-41 ff, Abgase durch Adsorption, beispielsweise an Aktivkohle, oder durch Absorption in einer Flüssigkeit gereinigt werden. Dabei entsteht aber das Problem der Entsorgung des beladenen Ad- bzw. Absorptionsmittels, zumal im vorliegenden Falle Produkte sehr unterschiedlicher chemischer und physikalischer Eigenschaften zu entfernen sind und eine der Substanzen von sicherheitstechnischer Relevanz ist und zu Verpuffungen oder Explosionen führen kann.

Erschwerend kommt hinzu, daß Kohlendioxid in großen Mengen, und zwar nach der oben angegebenen Gleichung in stöchiometrischen Mengen gebildet wird. Die Nutzung der physikalischen Löslichkeit von Ketonen und Dihydrofuranen reicht hier oft nicht aus, um die genannten Bestandteile ausreichend abzutrennen. Außerdem verbietet sich wegen der großen Mengen an Kohlendioxid eine Abtrennung durch wäßrig-alkalische Wäsche, da dabei erhebliche Salzmengen anfallen.

Das erfindungsgemäße Verfahren wird erfolgreich auch auf das Abgas der Reaktion, bei der man Cyclopropylalkylketone aus 3-Acyltetrahydrofuran-2-onen herstellt, angewandt: Dabei führt man die sauer katalysierte Umsetzung mit den Alkoholen, Wasser oder Alkohol/Wasser-Gemischen als Reaktivwäsche durch.

Der Überschuß an Waschflüssigkeit im Vergleich zu den abzutrennenden Verbindungen kann bei der Abgasreinigung beliebig hoch sein.

Vorzugsweise reinigt man das Abgas in zwei Stufen. In einer ersten Stufe wird dabei über 50 % des 4,5-Dihydro-2-alkylfurans mit Hilfe von Alkoholen, Wasser oder Alkohol/Wasser-Mischungen abgetrennt. In einer zweiten Stufe wird anschließend mit Schwefelsäure gewaschen.

Als Waschflüssigkeit der zweiten Stufe kommt nach dem erfindungsgemäßen Verfahren 60 bis 100 %ige, vorzugsweise 70 bis 100 %ige Schwefelsäure zum Einsatz.

Diese Schwefelsäure vermag aus dem Abgas der ersten Waschstufe die noch verbleibenden Mengen an Cyclopropylalkylketon und 4,5-Dihydro-2-alkylfuran praktisch quantitativ herauszuholen.

Unabhängig von den Gehalten an Abbauprodukten ausgewaschener organischer Verbindungen kann die Wasch-Schwefelsäure anschließend problemlos in einem üblichen Herstellungsprozeß zur Gewinnung von Schwefelsäure als Rohstoff eingesetzt und die enthaltene Schwefelsäure somit umweltschonend zurückgewonnen werden.

Die Reaktivwäsche mit dem Alkohol oder Alkohol/Wasser-Gemisch wird im allgemeinen bei 15 bis 70 °C, die Wäsche mit Schwefelsäure bei 10 bis 50 °C durchgeführt.

Durch das erfindungsgemäße Verfahren gelingt es, 4,5-Dihydro-2-alkylfurane sicher und unter kontrollierter Freisetzung der Reaktionswärme zu Additionsprodukten umzusetzen und praktisch quantitativ abzutrennen.

Die Entsorgung der bevorzugt eingesetzten organischen Medien kann unter Nutzung der thermischen Energie durch Verbrennung erfolgen. Da in diesem Falle dann an die Reinheit der eingesetzten Flüssigkeit keine hohen Ansprüche gestellt werden, können dafür auch geeignete Abfallprodukte aus anderen Produktionsprozessen, beispielsweise wirtschaftlich wenig attraktive, ggf. wasserhaltige Zwischemraktionen geeigneter Zusammensetzung verwendet werden.

Die folgenden Beispiele sollen die Erfindung verdeutlichen.

### Beispiel 1:

Ein Gemisch aus 97,0 g (1,15 mol) 4,5-Dihydro-2-methylfuran (DHMF), 483,6 g (5,75 mol) Cyclopropylmethylketon (CPMK) und 1,0 g Montmorillonit K 10 (Fa. Südchemie, D-80333 München) wird bei 25 °C gerührt. Man gibt langsam 71,5 g (1,15 mol) Ethylenglykol hinzu, wobei man eine Reaktionstemperatur von maximal 30 °C einhält. Man läßt 1 h nachrühren und filtriert dann den Katalysator ab. Das Gemisch wird mit 2 g Natriumcarbonat versetzt und anschließend über eine 0,5 m Multifil-Kolonne unter Normaldruck fraktioniert. Während der Destillation wird eine Sumpftemperatur von 200 °C nicht überschritten. Man erhält vorlauffrei 43,2 g (90 %) CPMK mit einer GC-Reinheit von > 99 %.

### Beispiel 2:

Ein Gemisch aus 195,0 g (2,3 mol) CPMK, 38,7.g (0,46 mol) DHMF und 1,0 g K10 wird bei 25 °C unter Rühren vorgelegt. Man dosiert 29,2 g (0,46 mol) Ethylenglykol hinzu, wobei die Temperatur bis auf 40 °C ansteigt. Man läßt 1 h nachrühren, filtriert den sauren Katalysator ab und gibt 2 g Natriumcarbonat zum Gemisch. Anschließend fraktioniert man das Gemisch über eine 0,5 m Multifil-Kolonne bei einem Druck von ca. 100 mbar. Bei einer Sumpftemperatur von maximal 80 °C erhält man vorlauffrei 168,5 g (85 %) CPMK mit einer GC-Reinheit von > 98 %.

### Beispiel 3:

In einem Reaktions-Kalorimeter (Mettler RC-1) wird bei 25 °C ein Gemisch aus 360,0 g Butanol und 40,0 g 10 %iger wäßriger Schwefelsäure unter Rühren vorgelegt. Mit einer Geschwindigkeit von 160,0 g/h wird DHMF für 0,5 h eingeleitet, wobei die entstehende Reaktionswärme/Zeit zu jeder Zeit mit der theoretisch zu erwartenden Wärmemenge/Zeit (unter adiabatischer Versuchsführung wurde vorher die Reaktionswärme der Reaktion von DHMF mit Butanol/10 % Schwefelsäure mit 40 kJ/mol bestimmt) verglichen wird. Beide Wärmemengen/Zeit (20 J/s) stimmen zu jedem Zeitpunkt überein. Bei der Einleitung von DHMF findet also eine momentane Reaktion von DHMF zu DHMF-Additionsprodukten statt.

### Beispiel 4:

In einem Reaktions-Kalorimeter (Mettler RC-1) werden bei 25 °C 400,0 g einer 50 %igen wäßrigen Essigsäure unter Rühren vorgelegt. Mit einer Geschwindigkeit von 160,0 g/h wird DHMF für 0,5 h eingeleitet, wobei die entstehende Reaktionswärme/Zeit zu jeder Zeit mit der theoretisch zu erwartenden Wärmemenge/Zeit (unter adiabatischer Versuchsführung wurde vorher die Reaktionswärme der Reaktion von DHMF mit 50 %iger Essigsäure mit 40 kJ/mol bestimmt) verglichen wird. Beide Wärmemengen/Zeit (20 J/s) stimmen zu jedem Zeitpunkt überein. Bei der Einleitung von DHMF findet also eine momentane Reaktion von DHMF zu DHMF-Additionsprodukten statt.

### Beispiel 5:

Ein Abgasstrom von 350 l/h Kohlendioxid mit einem Gehalt von 17 g/m³ CPMK und 34 g/m³ DHMF wird bei einer Temperatur von 25 °C am Fuß einer Kolonne mit einer Länge von 300 mm und einem Durchmesser von 30 mm eingeleitet. Die Kolonne besitzt eine 200 mm-Packung Raschigringe (Durchmesser 6 mm). Das Waschmedium, 260 g einer Mischung aus 2-Ethylhexanol mit 1,35 % Adipinsäure, wird über eine Umwälzpumpe mit einer Wälzrate von 12 l/h am Kolonnensumpf abgezogen und wieder auf den Kopf aufgegeben. Das die Waschkolonne verlassende Gasgemisch mit einer Beladung von 5,4 g/m³ CPMK und 11,2 g/m³ DHMF wird über eine Glasfritte in 500 g konzentrierte Schwefelsäure (100 %ig) eingeleitet.

Am Ausgang dieser zweistufigen Wäschekombination konnten über einen Zeitraum von 16 h keine organischen Bestandteile in der Gasphase nachgewiesen werden.

### Beispiel 6:

Der Versuchaufbau ist wie in Beispiel 5 angegeben, als Waschmedium für die erste Stufe werden jedoch 260 g einer Mischung aus Ethylenglykol mit 5 % p-Toluolsulfonsäure eingesetzt. Die Beladung nach dem ersten Wäscher beträgt 6,5 g/m³ CPMK und 6,7 g/m³ DHMF. Nach dem zweiten Wäscher ist wiederum keine Beladung nachzuweisen.

## Patentansprüche

1. Verfahren zur Herstellung von Cyclopropylalkylketonen mit C₁- bis C₄-Alkylgruppen aus 3-Acyltetrahydrofuran-2-onen,
**dadurch gekennzeichnet,**
**daß** man die dabei auch anfallenden 4,5-Dihydro-2-alkylfurane vor, während oder nach der Destillation der Reaktionsprodukte mit Alkoholen, Wasser oder Alkohol/Wasser-Gemischen bei 10 °C bis 80 °C in Gegenwart eines sauren Katalysators umsetzt und dann abtrennt.

2. Verfahren nach Anspruch 1,
**dadurch gekennzeichnet,**
**daß** das Gewichtsverhältnis von 4,5-Dihydro-2-alkylfuran zu Alkoholen, Wasser oder Alkohol/Wasser-Gemischen bei 1 : 2 bis 1 : 50 liegt.

3. Verfahren nach Anspruch 2,
**dadurch gekennzeichnet,**
**daß** ein Gewichtsverhältnis von 1: 5 bis 1 : 30 eingestellt wird..

4. Verfahren nach Anspruch 1,
**dadurch gekennzeichnet,**
**daß** die Alkohol/Wasser-Gemische einen Wassergehalt von bis zu 40 Gew.-% aufweisen.

5. Verfahren nach Anspruch 1,
**dadurch gekennzeichnet,**
**daß** die Alkohole einen Siedepunkt von 100 bis 300 °C aufweisen.

6. Verfahren nach Anspruch 1,
**dadurch gekennzeichnet,**
**daß** man bei der Herstellung von Cylcopropylmethylketon aus 3-Acetyltetrahydrofuran-2-on das 4,5-Dihydro-2-methylfuran abtrennt.

7. Verfahren nach Anspruch 1,
**dadurch gekennzeichnet,**
**daß** als Katalysatoren bis zu 15 Gew.-% Carbonsäuren, Sulfonsäuren oder Mineralsäuren, bezogen auf die Summe aus Alkoholen und Wasser, angewandt werden.

8. Verfahren nach Anspruch 7,
**dadurch gekennzeichnet,**
**daß** 1 bis 6 Gew.-% Carbonsäuren, Sulfonsäuren oder Mineralsäuren enthalten sind.

9. Verfahren nach Anspruch 1,
**dadurch gekennzeichnet,**
**daß** als Katalysatoren saure Ionenaustauscher oder saure Mineralien verwendet werden.

10. Verfahren nach Anspruch 1,
**dadurch gekennzeichnet,**
**daß** man die Umsetzung vor, während oder nach der Destillation der Reaktionsprodukte auf das Abgas anwendet und dabei eine Reaktivwäsche durchführt.

11. Verfahren nach Anspruch 10,
**dadurch gekennzeichnet,**
**daß** man in einer ersten Stufe über 50 % der 4,5-Dihydro-2-alkylfurane durch Reaktivwäsche mit Alkoholen oder Alkohol/Wasser-Mischungen entfernt und in einer zweiten Stufe mit 60 bis 100 %iger Schwefelsäure wäscht.

## Claims

1. A process for the preparation of cyclopropyl alkyl ketones containing C₁- to C₄-alkyl groups from 3 -acyltetrahydrofuran-2-ones, **characterized in that** the 4, 5-dihydro-2-alkylfurans also produced are reacted with alcohols, water or alcohol/water mixtures at from 10°C to 80°C in the presence of an acidic catalyst before, during or after distillation of the reaction products and are then separated off.

2. A process according to claim 1,
**characterized in that**
the weight ratio of 4,5-dihydro-2-alkylfuran to alcohols, water or alcohol/water mixtures is from 1:2 to 1:50.

3. A process according to claim 2,
**characterized in that**
a weight ratio of from 1:5 to 1:30 is established.

4. A process according to claim 1,
**characterized in that**
the alcohol/water mixtures have a water content of up to 40% by weight.

5. A process according to claim 1,
**characterized in that**
the alcohols have a boiling point of from 100 to 300°C.

6. A process according to claim 1,
**characterized in that**,
in the preparation of cyclopropyl methyl ketone, the 4,5-dihydro-2-methylfuran is separated off from 3 -acetyltetrahydrofuran-2-one.

7. A process according to claim 1,
**characterized in that**
the catalyst used is up to 15% by weight of carboxylic acids, sulphonic acids or mineral acids, based on the total amount of alcohols and water.

8. A process according to claim 7,
**characterized in that**
from 1 to 6% by weight of carboxylic acids, sulphonic acids or mineral acids are present.

9. A process according to claim 1,
**characterized in that**
the catalysts used are acidic ion exchangers or acidic minerals.

10. A process according to claim 1,
**characterized in that**
the reaction before during, or after distillation of the reaction products is preformed on the offgas and a reactive wash is thereby carried out.

11. A process according to claim 10,
**characterized in that**
in a first stage more than 50% of the 4,5-dihydro-2-alkylfurans are removed by reactive washing with alcohols or alcohol/water mixtures, and in a second stage they are washed with from 60 to 100% strength sulphuric acid.

## Revendications

1. Procédé de préparation de cyclopropylalkylcétones ayant des groupes alkyle en C₁ à C₄ à partir de 3-acyltétrahydrofuran-2-ones,
**caractérisé en ce qu'**
on fait réagir et ensuite on sépare des 4,5-dlhydro-2-alkylfuranes même qui précipitent, avant, pendant ou après la distillation des produits de réaction avec des alcools, de l'eau ou des mélanges alcool/eau entre 10°C et 80°C en présence d'un catalyseur acide.

2. Procédé selon la revendication 1,
**caractérisé en ce que**
le rapport en poids du 4,5-dihydro-2-alkylfurane aux alcools, à l'eau ou aux mélanges alcool/eau se situe à 1 : 2 à 1 : 50.

3. Procédé selon la revendication 2,
**caractérisé en ce qu'**
on ajuste un rapport en poids de 1 : 5 à 1 : 30.

4. Procédé selon la revendication 1,
**caractérisé en ce que**
les mélanges alcool/eau possèdent une teneur en eau allant jusqu'à 40 % en poids.

5. Procédé selon la revendication 1,
**caractérisé en ce que**
les alcools possèdent un point d'ébullition allant de 100 à 300°C.

6. Procédé selon la revendication 1,
**caractérisé en ce qu'**
au cours de la préparation de la cyclopropylméthylcétone, on sépare le 4,5-dihydro-2-méthylfurane de la 3-acétyltétrahydrofurane-2-one.

7. Procédé selon la revendication 1,
**caractérisé en ce que**
comme catalyseurs on utilise jusqu'à 15 % en poids d'acides carboxyliques, d'acides sulfoniques ou d'acides minéraux, rapportés à la somme d'alcool et d'eau.

8. Procédé selon la revendication 7.
**caractérisé en ce que**
de 1 à 6 % en poids d'acides carboxyliques. d'acides sulfoniques ou d'acides minéraux sont contenus.

9. Procédé selon la revendication 1,
**caractérisé en ce que**
comme catalyseurs on utilise des échangeurs d'ions acides ou des minéraux acides.

10. Procédé selon la revendication 1,
**caractérisé en ce qu'**
on utilise la réaction avant, pendant ou après la distillation des produits de réaction sur les gaz de combustion et on effectue ainsi un lavage réactif.

11. Procédé selon la revendication 10,
**caractérisé en ce qu'**
on élimine dans une première étape plus de 50% du 4,5-dihydro-2-alkylfurane par lavage réactif avec des alcools ou des mélanges alcool/eau et on lave dans une seconde étape avec de l'acide sulfurique à 60 à 100 %.
